# EUROPEAN PATENT SPECIFICATION

(11) **EP 0 265 399 B1**
(45) Date of publication and mention of the grant of the patent: **14.07.1993**
(21) Application number: 87830374.2
(22) Date of filing: 22.10.1987
(51) Int. Cl.: B29D 11/02, A61F 2/14

(54) **A process for the production of ocular prostheses, scleral lenses and contact-lenses, and the products thereof**
Verfahren zum Herstellen von Augenprothesen, sklerotischen Linsen und Kontaktlinsen, sowie nach dem Verfahren hergestellte Gegenstände
Procédé de fabrication de prothèses oculaires, de lentilles sclérotiques ou de contact et produits obtenus

(30) Priority: 23.10.1986 IT 4857886
(43) Date of publication of application: 27.04.1988
(73) Proprietor: Del Re, Francesco, I-00195 Rome (IT)
(72) Inventor: Del Re, Francesco, I-00195 Rome (IT)
(74) Representative: de Simone, Domenico

(56) References cited:
- DE-A- 3 024 795
- GB-A- 2 055 584
- US-A- 2 433 261
- US-A- 2 603 791
- US-A- 2 817 845

## Description

The present invention relates to a process for the production of ocular prostheses, scleral lenses and contact-lenses, and to the products obtained thereby.

More particularly, the present invention relates to a process for the obtainment of ocular prostheses from orbital mold, as performed prostheses or as prostheses that can be adapted to any type of surgical implantation, and for the obtainment of preformed scleral lenses or of scleral lenses form orbital mold, and of cosmetic contact-lenses in which recourse is had to photographic means for the realization of the cosmetic part.

As well known, the cosmetic part both of prostheses and lenses, either contact-lenses or scleral lenses, has been realized previously by hand painting of the iris and of the pupil, in association with silk-screen processes.

More particularly, the iris was painted on the scleral or on the lenses chosen by the patient, employing different types of coloring agents or dyes or pigments, and next performing the various polymerization operations that are necessary for obtaining the complete final article.

This classical technology compulsorily required the continual presence of skilled personnel, especially for the reproduction by painting of the patients' irises, said patients being forced to a very heavy repeated cycle of visits.

In addition, the result of a reproduction of an iris by painting, as accurate as it may be, does not allow a precise fidelity of reproduction of the actual iris to be warrented, both as regards the color and as regards the size.

Such drawbacks, together with the very high cost of the finished article, are on the whole very heavy in the case in which the ocular prostheses is to be substituted during the years for adaptation to changes in sizes of the ocular hollowness, as occurs in the case of children, or otherwise for adaptation to the chromatic changes in the iris itself.

It is to be remarked that the whole of the operations that are traditionally required for carrying out the necessary technology, comprises as its own feature a handicraft character that opposes the realization of an industrial activity addressed to the making of ocular prostheses of the kind mentioned above, so that a patient must necessarily apply to the few handicraft centers available.

Accordingly, it is well clear that there was the need for a process for the realization of ocular prostheses, scleral lenses, and contact-lenses, which process allows the drawbacks mentioned above to be fully removed or at least reduced, by directing the relative technology towards solutions of industrial character.

Thus, in US-A-2,603,751 an artificial eye is disclosed, having an iris comprising a plurality of superimposed, differently colored positive and negative photographic images of a human eye, said images being spaced apart in parallel planes with transparent plastic material therebetween.

Therefore, this document avoides the drawbacks connected with hand parting of the iris and although it constitutes a progress in the art, however does not overcome other drawbacks, such as remarkable differences between the iris of the patient and the artificial iris consisting of a plurality of superimposed, differently colored positive and negative photographic images of a human eye, which could not be exactly positioned with respect to one another and on the prosthasis, beside that the arrangement thereof is very intricated and thus expensive and hardly could give an impression of a natural eye.

The more recent DE 3024795 AI likely relates to an artificial eye having a photographic image of a human iris and although the artificial eye suggested in this German document is simplier with respect to the above mentioned US patent, the arrangement of the photographic image on its support required an intricated process. Thus, the photographic image is directly inserted between two convex respectively concave surfaces of two cup shaped elements and assumes, moreover, an arcuated shape, in opposition to the flat confirmation of a natural human iris. Furthemore, there is not provided a capsule shaped portion simulating the crystalline lens of a human eye. Thus, the artificial eye has not the look of an actual human eye. The artificial eye of this German document has also a further drawback consisting in that is does not provide means for exactly positioning the photographic image on the prostheses.

Thus, it is an object of the present invention provide an artificial eye having single that photographic image of iris incorporated within a button shaped circular element having a transparent, crystalline lens simulating outer layer which forms with said circular element a capsule having means for achieving a precise alignment and exact positioning of the iris image bearing capsule within the prostheses.

The photographic reproduction of iris is the most delicate operation, that requires some particular techniques, especially for eliminating the corneal light reflections which would alter the real image of the iris.

Indeed, it is necessary to eliminate the reflections of the lachrymal film which interfere with the realization of a correct photograph of the iris.

To that aim, the technology used by the present invention is based on screening systems applied to the patient, such as opaque black spectacles having diameters slightly larger than the diameter of the iris, or to opaque-black lenses bearing a central hole of diameter again larger than that of the iris, or otherwise to external screening systems.

In order to obtain the necessary reproduction fidelity, some special techniques are to be followed which are fundamental in the photographic step and development, which techniques also require the availability of contact printing means with the electronic color analyzer.

According to the most general realization of the process of the present invention, said process comprises the making of a correct photograph of the iris, its application to a base support, the coating with a clear polymeric material and next the shaping till the realization of a capsule in the shape of a button which is suitable for being encased or inserted into an ocular prosthesis or a scleral lens or a contact-lens, and has means for achieving a precise alignment and exact positioning of said capsule containing the photographic image of the iris within the protheses.

Thus, the present invention relates to a process for producing ocular prostheses and scleral lenses including the step of photographing the iris of the patient eye; punching the photographic image to the desired size and placing said photographic image onto a flat support lamina, in which:
- the iris assembly formed by said support lamina and said photographic iris image is placed within a first mold, into which a transparent acrylic monomer compound is poured to form a button shaped element consisting of an outer acrylic layer and said iris assembly;
- the outer face of said button is turned to obtain a bulb-like capsule provided with a central protrusion;
- said bulb like capsule is placed into a second mold having a central cavity by inserting said protrusion into said cavity, pouring into said second mold a mixture of an acrylic monomer compound and pigments and polymerizing said mixture to attain a sclera portion of a so obtained ocular prosthesis;
- removing the acrylic polymer layer from the surface of said capsule excluded the border portion between said sclera and said outer layer;
- exerting on said border portion a light eroding action and
- coating the entire surface of said sclera, said eroded border portion and said outer layes with a layer of a transparent acrylic material.

Preferably, methylmethacrylate is employed as the acrylic monomer, for instance the product is employed which is commercially available with the registered trade mark "Orthocryl" from Dentaurum.

The polymerization reaction is carried out advantageously by heating to about 110°C in the presence of free radicals catalysts (as for instance benzoyl peroxide), keeping the pressure above the atmospheric pressure just in order to warrant the correct positioning of the associated members.

It is to be remarked that during the turning operation advantageously it is preferable to limit the extent of the convex of bulging shape to the upper superficial portion of the button, which will show a slight slope to the vertical at the points corresponding to the lateral surface of the same.

The process according to the present invention which is directed to the production of scleral lenses is substantially identical with the process already described for obtaining the prostheses; the only difference being in that the realization is to be obtained of variable bend parallel faces, said bend being variable according to the application requirements.

Substantially the process for the making of scleral lenses is characterized in that the button containing the photograph of the iris is to be placed into the central portion of the lens mold, said portion bearing a small cavity into which the protruding part of said button is to be inserted, then a mixture of an acrylic monomeric compound and of a a free radical catalyst is introduced, and a cold- or a hot-polymerization is carried out till the scleral lens is obtained; the layer of the acrylic polymer is then removed from the most part of the outer surface of the button, excluding the two border portions between the button and the lens, exerting a less eroding action on such portions, and causing a gradual change to occur between the chromatic shades of the button and those of the lens.

It is to be remarked that in such case the button containing the photograph of the iris is to be of reduced thickness which is limited by surface portions having different bends, as the inside surface is to correspond to lens base curvature whereas the outer surface is to correspond to the anatomical profile of the iris.

The polymerization ways adopted are also similar to those adopted in the procedure for the making of ocular prostheses.

When wishing to give the scleral lens a dioptrical power, the scleral lens is pierced at the point corresponding to the pupil through its full thickness, such hole being filled with a material and the lens being turned over its outside surface.

The same kind of technology based on the photographic reproduction can be adopted for the production of cosmetic contact-lenses, possibly having incorporated a dioptrical power.

The products deriving from the application of the procedures mentioned above and, more particularly, the ocular prostheses, both of the preformed type and from orbital mold or those which can be adapted to any type of surgical implantation, the scleral lenses of the preformed type or from orbital mold and cosmetic contact-lenses, all belong within the scope of the priority rights of the present invention.

The present invention will be disclosed in the following with reference to some particularly preferred embodiments which are illustrated in the enclosed drawings, wherein:
Figure 1 shows an exploded view of the button containing the photographs of the iris according to the present invention;
Figure 2 shows a cross sectional view of the closure button 1;
Figure 3 shows a front view of the sclera, on which the final position is pointed out of the button containing the photograph of the iris;
Figure 4 shows a view of the sclera of Figure 3 with the button applied;
Figure 5 shows a cross sectional view along the line 5-5 of Figure 4, and
Figures 6 and 7 show a scleral lens before the application of the button.

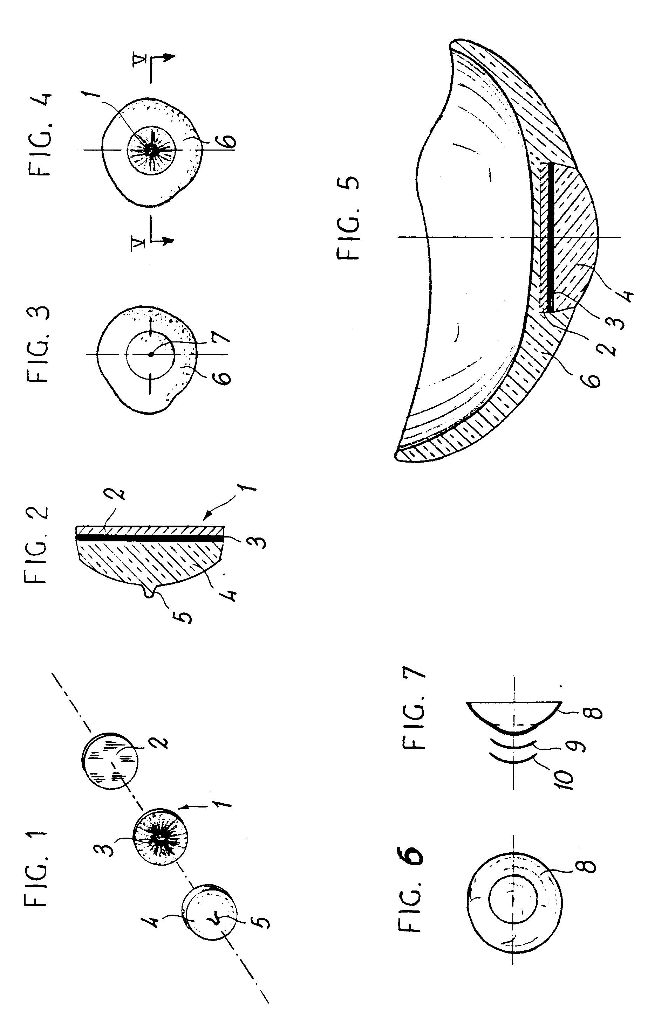

As can be observed in Figure 1, the button 1 is made up of a base lamina 2, a photograph of the iris 3 suitably punched and of an outer layer 4 of acrylic material provided with a protruding part 5 at the point corresponding to the center of its top surface.

Such details also can be observed in Figure 2, which illustrates better the particular shape of the outer layer 4, especially on the lateral surface which is at a slight slope to the vertical.

In Figures 3 and 4 the ocular sclera 6 can be observed on which (Figure 3) the seat or housing is drawn which is designed to house the button 1. A cavity is realized in the respective mold at a point corresponding to the center 7 of the sclera 6, which cavity is designed to house said protruding part 5 so as to warrant an accurate and sure positioning.

Figure 5 shows the complete ocular prosthesis in which the button 1 is illustrated, that is made up of the members 2, 3 and 4 and is made integral with the sclera following the polymerization of the acrylic material which it consists of.

It is to be remarked that the configuration of the lateral surface of the layer 4 makes it possible to show the original ocular limbus.

Figures 6 and 7 finally illustrate a scleral lens 8 on which the photograph and the outer acrylic coating 10 are applied and correctly positioned at the central position.

The present invention has been disclosed with reference to a specific embodiment of the same, but it is to be understood that modifications and changes can be introduced in the same by those who are skilled in the art without departing from the scope of the invention for which a priority right is claimed.

## Claims

1. A process for producing ocular prostheses and scleral lenses including the step of photographing the iris of the patient eye; punching the photographic image (3) to the desired size and placing said photographic image (3) onto a flat support lamina (2), characterized in that:
- the iris assembly formed by said support lamina (2) and said photographic iris image (3) is placed within a first mold, into which a transparent acrylic monomer compound is poured to form a button shaped element consisting of an outer acrylic layer (4) and said iris assembly (2, 3);
- the outer face of said button is turned to obtain a bulb-like capsule provided with a central protrusion (5);
- said bulb-like capsule is placed into a second mold having a central cavity by inserting said protrusion (5) into said cavity, pouring into said second mold a mixture of an acrylic monomere compound and pigments and polymerizing said mixture to attain a sclera (6) portion of a so obtained ocular prosthesis;
- removing the acrylic polymer layers from the surface of said capsule (2, 3, 4), excluded the border portion between said sclera (6) and said outer layer (4);
- exerting on said border portion a light eroding action and
- coating the entire surface of said sclera (6), said eroded border portion and said outer layer (4) with layer of a transparent acrylic material.

2. A process for the production of ocular prostheses and scleral lenses according to claim 1, said process being characterized in that said acrylic monomer is the monomer known as "Orthocryl" available from Dentaurum.

3. A process for the production of ocular prostheses and scleral lenses according to claims 1 and 2, characterized in that said polymerization reaction is carried out by heating to about 100°C in the presence of free radicals catalysts at a pressure higher than the atmospheric pressure.

4. A process for the production of ocular prostheses and scleral lenses according to claim 1, said process being further characterized in that during said turning operation of the outer layer (4) of said capsule the extension of the convex or bulging shape is limited to the upper superficial portion of the capsule itself, which surface is at a slight slope to the vertical at the points corresponding to its lateral surface.

## Patentansprüche

1. Verfahren zur Herstellung von Augenprothesen und sklerotischen Linsen, wobei der Iris des Patientenauges photographiert, die photographische Aufnahme (3) in gewuenschten Groesse ausgestanzt und auf einer flachen Trageplatte (2) angeordnet wird, dadurch gekennzeichnet:
- dass die durch die vorgenannte Trageplatte (2) und die vorgenannte photographische Irisaufnahme gebildete Iriseinheit in eine erste Form eingefuehrt wird, in welche ein durchsichtiges Akrylmonomer eingegossen wird, um einen knopfartigen, aus einer akrylischen Aussenschicht (4) und der vorgenannten Iriseinheit (2,3) bestehenden Teil zu formen;
- dass die Aussenflaeche des genannten knopfartigen Teiles abgedreht wird, um eine augapfelartige Kapsel mit einem mitteren Vorsprung zu bilden;
- dass die genannte augapfelartige Kapsel ein eine zweite eine mittere Aushoehlung aufweisende Form eingefuehrt wird, wobei der genannte Vorsprung (5) in diese Aushoehlung eingesteckt wird, und in diese zweite Form ein aus einer akrylischen Monomerverbindung und Pigmenten bestehendes Gemisch eingegossen und dann polymerisiert wird, um so einen Sklerateil des so gebildeten Augnphothesis zu formen;
- dass die akrylische Polymerschicht von der Oberflaeche der genannten Kapsel (2,3,4) entfernt wird, mit Ausnahme der Randteile zwischen dem genannten Sklerateil (6) und der genannten Aussenschicht (4);
- dass auf die genannten Randteile eine leichte Aetzwirkung ausgeuebt wird und
- dass die ganze Oberflaeche des genannten Sklerateiles (6), des genannten Randteiles und der genannten Aussenschicht (4) mit einer Schicht aus einem durchchsichtigen Akrylharz ausgekleidet wird.

2. Verfahren zur Herstellung von Augenprothesis und sklerotischen Linsen nach Anspruch 1, dadurch gekennzeichnet, dass das genannte Akrylmonomer ein von Dentaurum erhaeltliches und unter dem Namen "Orthocryl" vertiebenes Monomer ist.

3. Verfahren zur Herstellung von Augenprothesis und sklerotischen Linsen nach Anspruch 1 und 2, dadurch gekennzeichnet, dass die genannte Polymerizationsreaktion durch Erwaermen auf etwa 100.C in Anwesenheit von Freiradikale-Katalizzatoren unter einem von atmosphaerischen Druck hoeheren Druck durchgefuehrt wird.

4. Verfahren zur Herstellung von Augenprothesis und sklerotischen Linsen nach Anspruch 1, dadurch gekennzeichnet, dass waehrend des genannten Drehens der Aussenschicht (4) der genannten Kapsel, die Groesse der konvexen oder gewoelbten Form auf den oberen Flaechenteil der Kapsel beschraenkt wird, der gegenueber der Vertikallinie an den seiner Seitenflaeche entsprechenden Stellen leicht geneigt ist.

## Revendications

1. Procédé de fabrication de prothèses oculaires et de lentilles sclératiques, comprenant la phase de photographier l'oil de patient, la phase de poinçonner l'image photographique (3) à dimension desirée et la phase de placer ladite image photographique (3) sur un support à lame plate (2) caractérizé en ce que:
- l'ensemble d'iris, formé par ledit support à lame (2) et ladite image photographique de l'iris, est placé dans une premiere forme dans laquelle est versé un monomère acrylique transparent pour former un élément à bouton consistant d'une couche acrylique extérieure (4) et dudit ensemble d'iris (2, 3);
- la face extérieur dudit bouton est tournée pour former une capsule à bulbe munie d'une saillie central (5);
- ladite capsule à bulbe est placée dans une deuxieme forme ayant une cavité centrale par insertion de ladite saillie (5) dans ladite cavité, et dans laquelle est versé un mélange d'une composition monomère acrylique et de pigments, qui est polymérisé pour former une partie sclérotique d'une prothèse oculaire ainsi obtenue;
- lesdites couches de polymère acrylique sont enlevées de la surface de ladite capsule (2, 3, 4), à l'exception de la partie de bord entre ladite partie sclérotique (6) et ladite couche extérieure (4);
- sur ladite partie de bord une faible action d'érosion est exercée et
- la surface entière de ladite partie sclérotique (6), ladite partie de bord erosée et ladit couche extérieure (4) sont revêtues d'une couche d'un matérial acrylique transparent.

2. Procédé de fabrication de prothèses oculaires et de lentilles sclérotique selon la revendication 1, caractérisé en ce que ledit monomère acrylique est un monomère connu comme "Orthocryl" disponible chez Dentaurum.

3. Procédé de fabrication de prothèses oculaires et de lentilles sclérotique selon les revendication 1 et 2, caracterisé en ce que ladite rèaction de polymerérisation est effectuée par chauffage à peu près à 100°C en présence de cataliseurs à radicals libres et à une pression supérieure à la pression atmosphérique.

4. Procédé de fabrication de prothèses oculaires et de lentilles sclérotique selon la revendication 1, caracterisé en autre en ce que pendent ladite opération de tournage de la couche extérieure (4) de ladite capsule, l'étendue de la forme convex ou arquée est limité à la partie superficielle supérieure de la capsule, ladite surface ayant une inclinaison ou regard de la verticale aux points correspondants à sa surface latérale.
